# EUROPÄISCHE PATENTSCHRIFT

(11) **EP 1 285 912 B1**
(45) Veröffentlichungstag und Bekanntmachung des Hinweises auf die Patenterteilung: **28.12.2005**
(21) Anmeldenummer: 02016708.6
(22) Anmeldetag: 26.07.2002
(51) Int. Cl.: C07C 403/08, C07D 317/64

(54) **Verfahren zur selektiven Reduktion von Alkinverbindungen**
Process for the selective reduction of alkynic compounds
Procédé pour l'hydrogénation sélective de composés alkyniques

(30) Priorität: 22.08.2001 DE 10140180
(43) Veröffentlichungstag der Anmeldung: 26.02.2003
(73) Patentinhaber: BASF Aktiengesellschaft, 67056 Ludwigshafen (DE)
(72) Erfinder: Grimmer, Johannes, 67061 Ludwigshafen (DE); Müller, Thomas, Dr., 67246 Dirmstein (DE); Bomm, Volker, Dr., 67125 Dannstadt-Schauernheim (DE)

(56) Entgegenhaltungen:
- EP-A- 0 005 748

## Beschreibung

Die vorliegende Erfindung betrifft ein neues Verfahren zur Reduktion von Alkinverbindungen, insbesondere betrifft die Erfindung ein Verfahren zur Herstellung von Cyclohexenderivaten, die als Zwischenprodukte für die Herstellung von Carotinoiden geeignet sind.

Eine Vielzahl der in der Literatur beschriebenen technischen Carotinoid-Synthesen, u.a. die Herstellung von Astaxanthin, verläuft über Cyclohexen-Zwischenprodukte, die neben einer oder mehreren C=C-Doppelbindungen auch eine C≡C-Dreifachbindung enthalten. Zur Ausbildung eines konjugierten Doppelbindungssystems muß diese Dreifachbindung in einem separaten Verfahrensschritt partiell reduziert werden.

Dies kann im Rahmen der in DE-A-43 22 277 beschriebenen Astaxanthin-Synthese im Falle des Alkindiols IVa mit Zink/Essigsäure in Methylenchlorid erfolgen.

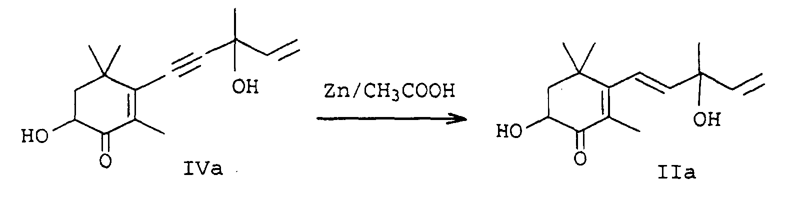

EP-A-0 005 748 betrifft ein weiteres Verfahren zur Herstellung von Astaxanthin, in der die partielle Reduktion des Alkindiols der Formel IIIa ebenfalls mit Zink/Essigsäure in Methylenchlorid durchgeführt wird.

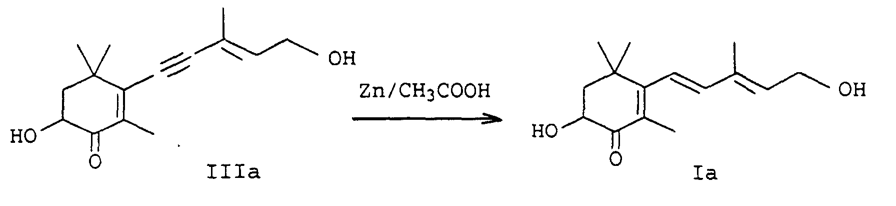

Nachteilig an der beschriebenen Zink/Essigsäure-Reduktion ist die unzureichende Selektivität der Methode. Unerwünschte Nebenprodukte wie z.B. die Bildung von Spiroverbindungen, die sich im weiteren Syntheseverlauf nicht in die gewünschten Folgeprodukte überführen lassen, können zu signifikanten Ausbeuteverlusten führen.

EP-A-1 197 483 beschreibt ein Verfahren zur katalytischen Reduktion von Alkinverbindungen unter Verwendung eines Gemisches aus Zink und mindestens einem Ammonium-, Kupfer-, Alkali- oder Erdalkalimetallsalz.

Weitere Reduktionsverfahren sind u.a. in J. Amer. Oil Chem. Soc. 49 (1972) 72 beschrieben, in der die Reduktion von Dreifachbindungen zu cis-Doppelbindungen in langkettigen, konjugierten Fettsäuren mit Zink in siedenden protischen Lösungsmitteln erfolgt.

Die hier genannten drastischen Reduktionsbedingungen sind für thermisch labile Verbindungen nicht geeignet.

In Helv. Chim. Acta 58 (1975) 1016 ist die Reduktion von konjugierten Alkinen in protischen Lösungsmitteln beschrieben. Als Reduktionsmittel verwenden die Autoren Zinkstaub, der durch Zugabe von Kaliumcyanid aktiviert wurde.

Die o.g. Methoden liefern einerseits nur mäßige Ausbeuten, die Aktivierung mit Kaliumcyanid führt andererseits zu einem beträchtlichen Gesundheitsrisiko.

Die Veröffentlichung im Journal für praktische Chemie 336 (1994) 714-715 beinhaltet eine Methode zur (Z)-selektiven Reduktion von konjugierten Dreifachbindungen mit einer Kombination aus Zn (Cu/Ag) in polaren protischen Lösungsmitteln wie z.B. Methanol/Wasser.

Dies Verfahren hat den Nachteil, dass die Herstellung des Reagenzes sehr aufwendig ist und zudem das Reagenz stets frisch hergestellt werden muss.

Es war daher die Aufgabe der vorliegenden Erfindung, ein Verfahren zur partiellen Reduktion von Alkinverbindungen bereitzustellen, mit dem die oben genannten Nachteile des Standes der Technik vermieden werden.

Diese Aufgabe wurde gelöst durch ein Verfahren zur Herstellung von Cyclohexenderivaten der allgemeinen Formeln I oder II, 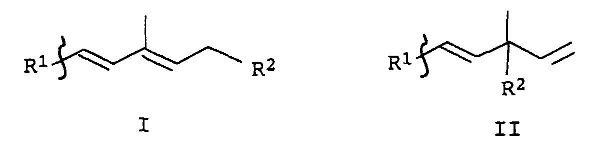 in der die Substituenten R¹ und R² unabhängig voneinander folgende Bedeutung haben:
- R¹: 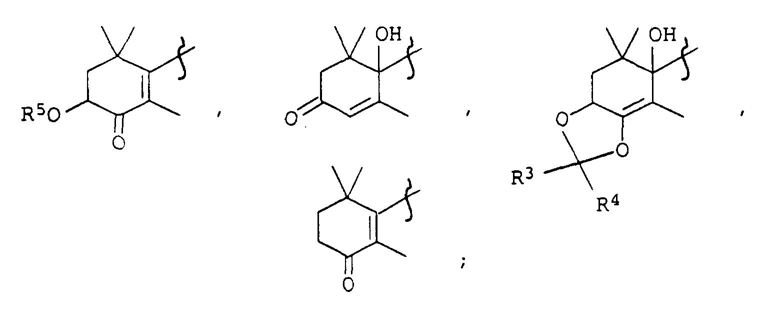
- R²: OH oder eine durch Hydrolyse in eine Hydroxygruppe überführbare Schutzgruppe,
- R³ und R⁴: Wasserstoff, C₁-C₄-Alkyl;
- R⁵: Wasserstoff, C₁-C₄-Acyl;
durch Reduktion von Alkinverbindungen der allgemeinen Formeln III oder IV, 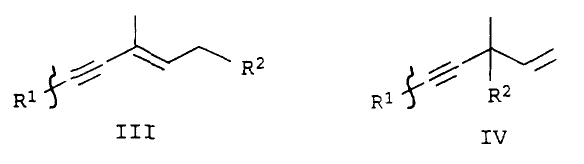 in der die Substituenten R¹ und R² die oben genannte Bedeutung haben, dadurch gekennzeichnet, dass man als Reduktionsmittel mindestens ein Salz der Dithionigen Säure, der Hydroxymethansulfinsäure oder ein Gemisch aus mindestens einem Salz der Dithionigen Säure und mindestens einem Salz der Hydroxymethansulfinsäure verwendet.

Als Alkylreste für R³ und R⁴ seien lineare oder verzweigte C₁-C₄-Alkylketten, z.B. Methyl, Ethyl, n-Propyl, 1-Methylethyl, n-Butyl, 1-Methylpropyl-, 2-Methylpropyl sowie 1,1-Dimethylethyl genannt. Bevorzugte Alkylreste sind Methyl und Ethyl.

Als Substituenten für R⁵ seien lineare oder verzweigte C₁-C₄-Acyl-ketten, z.B. Formyl, Acetyl, Propionyl, Isopropionyl genannt. Bevorzugter Acylrest ist Acetyl.

Unter einer durch Hydrolyse in eine Hydroxygruppe überführbare Schutzgruppe für R² kommen solche funktionellen Gruppen in Betracht, die relativ leicht in die Hydroxygruppe überführt werden können. Genannt seien beispielsweise Ethergruppen, wie 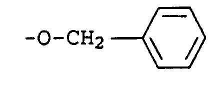 und -O-C(CH₃)₃,

Silylethergruppen, wie -O-Si(CH₃)₃, -O-Si(CH₂CH₃)₃, -O-Si(isoPropyl)₃, -O-Si(CH₃)₂(tert.-Butyl) und -O-Si(CH₃)₂(n-Hexyl) oder substituierte Methylethergruppen, wie die α-Alkoxy-alkylethergruppen der Formeln -O-CH₂-O-CH₃, 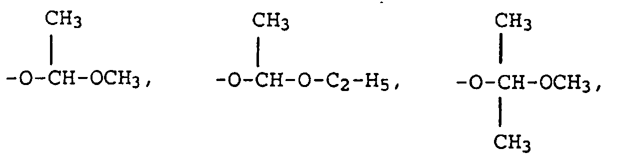 und geeignete Pyranylethergruppen, wie die Tetrahydropyranyloxygruppe und die 4-Methyl-5,6-dihydro-2H-pyranyloxy-Gruppe.

Mit besonderem Vorteil verwendet man für R² die Tetrahydropyranyloxygruppe 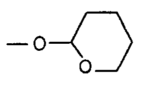 oder die α-Ethoxy-ethoxygruppe der Formel

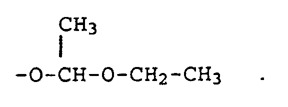

Bedingungen zur Abspaltung der o.g. Schutzgruppen finden sich u.a. in T. Greene "Protective Groups in Organic Chemistry", John Wiley & Sons, 1981, Chapter 2.

Als Salze der Dithionigen Säure sind im Rahmen der vorliegenden Erfindung Verbindungen der allgemeinen Formel M^{I}₂(O₂S-SO₂) zu verstehen, in denen M^{I} ein einwertiges Metall bedeutet. Bevorzugt handelt es sich hierbei um Natriumdithionit, Kaliumdithionit oder Zinkdithionit. Als besonders bevorzugtes Salz aus dieser Gruppe sei Natriumdithionit genannt.

Als Salze der Hydroxymethansulfinsäure [HO-CH₂-S(=O)OH] kommen bevorzugt Natriumformaldehydsulfoxylat oder Zinkformaldehydsulfoxylat, insbesondere Natriumformaldehydsulfoxylat in Frage.

Eine bevorzugte Ausführungsform des erfindungsgemäßen Verfahrens ist dadurch gekennzeichnet, dass man als Reduktionsmittel ein Gemisch aus mindestens einem Salze der Dithionigen Säure und einer Base, ausgewählt aus der Gruppe, bestehend aus Natriumcarbonat, Natriumhydrogencarbonat, Kaliumcarbonat und Kaliumhydrogencarbonat verwendet. Als besonders vorteilhaft hat sich als Reduktionsmittel die Kombination aus Natriumdithionit und NaHCO₃ erwiesen.

Blankit® (Fa. BASF Aktiengesellschaft), ein Reduktionsbleichmittel auf Basis Natriumdithionit und Sulfinsäure eignet sich ebenfalls.

Darüber hinaus kann das folgende Reduktionsmittel zur Anwendung kommen:
- Calciumhydroxymethansulfinat,

Gegenstand der Erfindung ist ebenfalls ein Verfahren zur Herstellung von Cyclohexenderivaten der Formel I oder II, dadurch gekennzeichnet, dass die Bildung von Natriumdithionit in situ z.B. nach dem sogenannten Formiatverfahren erfolgt, indem man Natriumformiat mit Natriumhydrogensulfit in 80 %igem Methanol reagieren lässt, danach durch Zugabe einer Base den pH-Wert auf ca. 9,5 einstellt und anschließend das zu reduzierende Agens in das Reaktionsgemisch einträgt.

Die in situ Bildung von Natriumdithionit kann auch über das sogenannte Borol-Verfahren durch Umsetzung von Natriumboranat mit Natronlauge und Schwefeldioxid erfolgen

Es hat sich gezeigt, dass die erfindungsgemäße Reduktion besonders gut in Gegenwart von Wasser erfolgt. Die Wassermenge wählt man so, dass die Base und das Reduktionsmittel gelöst vorliegen. In der Regel werden pro Mol eingesetzter Base und pro Mol Reduktionsmittel insgesamt 600 bis 4000 ml, besonders bevorzugt 800 bis 1600 ml Wasser verwendet.

Als weiteren Vorteil für den Verlauf der Reduktion hat sich die Zugabe eines inerten Lösungsmittels herausgestellt.

Als inertes Lösungsmittel kommen bei den erfindungsgemäßen Verfahren generell alle für die Verbindungen I bis IV inerten Solventien in Betracht. Vorzugsweise arbeitet man in chlorierten Kohlenwasserstoffen, wie z.B. in Dichlormethan, Perchlorethylen oder Chloroform, oder in einem etherischen Lösungsmittel, wie Dialkylethern, Tetrahydrofuran oder Dioxan, insbesondere in dem mit Wasser nicht mischbaren Methyl-tert.-butylether. Als weitere Lösungsmittel kommen auch aromatische Kohlenwasserstoffe, insbesondere Toluol sowie C₁-C₃-Alkohole, wie Methanol, Ethanol oder Propanol in Frage.

Vorzugsweise wird eine 10 bis 70 Gew.-%ige Lösung des Alkindiols in einem der o.g. Lösungsmittel, besonders bevorzugt eine 30 bis 65 Gew.-%ige Lösung des Alkindiols in Methylenchlorid verwendet.

Die Reduktion läuft in einem pH-Bereich von 6,0 bis 10, bevorzugt von 7,8 bis 9 ab.

Das eingesetzte Natriumdithionit wird in einer Menge von etwa 1,1 bis 5, bevorzugt von 1,5 bis 4, besonders bevorzugt von 2,1 bis 3 Grammatom pro Mol des zu reduzierenden Alkindiols eingesetzt. Um den pH-Bereich von 6,0 bis 10 während des Reduktionsverlaufs einhalten zu können, werden in Bezug auf 1 Mol Natriumdithionit 0,1 bis 4 Mol Base, bevorzugt 1 bis 3,5 Mol Base eingesetzt.

Die Reduktion kann bei Temperaturen zwischen Raumtemperatur und dem Siedepunkt des verwendeten Lösungsmittels durchgeführt werden.

Bevorzugte Reaktionstemperaturen liegen im Bereich von 30 bis 100°C, besonders bevorzugt im Bereich von 40 bis 80°C.

Eine besonders bevorzugte Verfahrensvariante des erfindungsgemäßen Verfahrens ist dadurch gekennzeichnet, dass man die Reduktion kontinuierlich durchführt, beispielsweise indem man das Reaktionsgemisch vorzugsweise bestehend aus Alkindiolen der Formeln IIIa oder IVa, einem organischen Lösungsmittel, Wasser, einer Base und Reduktionsmittel in einem Rohrreaktor oder Rührkesselkaskade bei erhöhter Temperatur im Bereich von 30 bis 100°C umsetzt.

Das erfindungsgemäße Verfahren ist insbesondere zur Herstellung der Cyclohexenverbindungen der Formeln Ia und IIa geeignet:

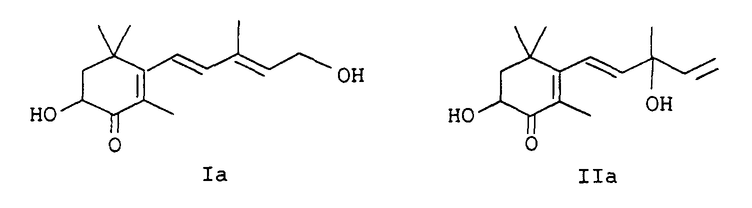

Bei der Durchführung des Verfahrens wird im allgemeinen so gearbeitet, dass man der Reihenfolge nach Wasser, Base und Alkinverbindungen (im Falle der Cyclohexenverbindungen der Formeln Ia und IIa verwendet man Alkinverbindungen der Formel IIIa oder IVa, gelöst in einem inerten Lösungsmittel) 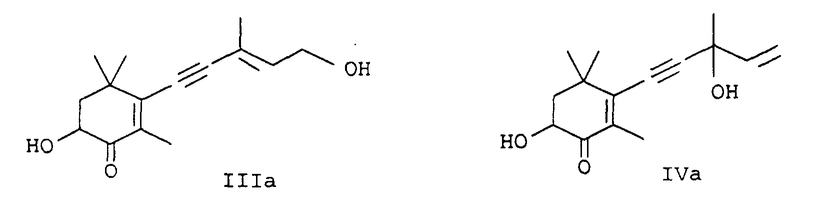 vorlegt und zum Schluss das Reduktionsmittel entweder portionsweise oder auf einmal einträgt.

Anhand der folgenden Beispiele soll der Gegenstand der vorliegenden Erfindung näher erläutert werden.

### Beispiel 1

Eine Lösung von 14,6 g NaHCO₃ in 225 ml Wasser wurde mit 12,5 g (0,05 Mol) 6-Hydroxy-3-(3-hydroxy-3-methyl-4-penten-1-inyl)-2,4,4-trimethyl-2-cyclo-hexen-1-on der Formel IVa mit einer Reinheit von 92 %, gelöst in 15 ml Methylenchlorid, versetzt und unter Rühren auf 35°C erwärmt. Anschließend wurden in das Reaktionsgemisch 21,8 g (0,125 mol) Natriumdithionit eingetragen und bei 50°C weitere 50 Minuten gerührt. Nach Abkühlung auf 20°C wurde das Reaktionsgemisch mit 100 ml Methylenchlorid versetzt, die wässrige Phase abgetrennt und die organische Phase 2 x mit 100 ml Wasser gewaschen, über Natriumsulfat getrocknet, abfiltriert und der Rückstand 2 x mit je 100 ml Methylenchlorid gewaschen. Nach Abdestillation des Lösungsmittels erhielt man einen öligen Rückstand, der nach gaschromatographischer Analyse 78,3 GC-Fl-% an Alkendiol der Formel IIa enthielt.

### Beispiel 2

Eine Mischung aus 200 ml Wasser, 24,6 g (0,293 mol) NaHCO₃, 25 g (0,1 Mol) Alkindiol der Formel IVa (Reinheit: 92 %ig) in 30 ml Methylenchlorid, 100 ml Methanol und 38,36 g (0,22 mol) Natriumdithionit wurde durch einen auf 60 bis 65°C vorgewärmten Rohrreaktor gepumpt. Der Auslauf am Reaktorende wurde in ein vorgekühltes Auffanggefäß geleitet und die daraus entnommenen Proben gaschromatographisch analysiert. Nach GC erhielt man 30,5 Fl.-% Alkindiol der Formel IVa und 66,7 Fl.-% Alkendiol der Formel IIa.

## Patentansprüche

1. Verfahren zur Herstellung von Cyclohexenderivaten der allgemeinen Formeln I oder II, 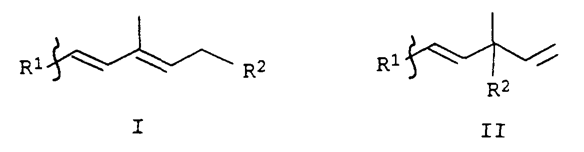 in der die Substituenten R¹ und R² unabhängig voneinander folgende Bedeutung haben:
R¹ 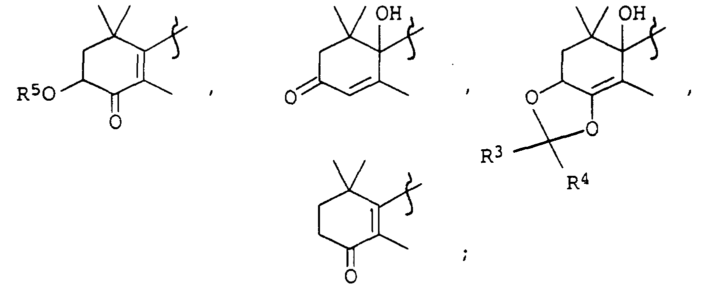
R² OH oder eine durch Hydrolyse in eine Hydroxygruppe überführbare Schutzgruppe,
R³ und R⁴ Wasserstoff, C₁-C₄-Alkyl;
R⁵ Wasserstoff, C₁-C₄-Acyl;
durch Reduktion von Alkinverbindungen der allgemeinen Formeln III oder IV, 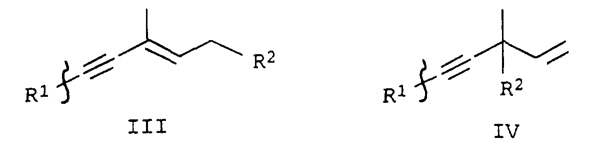 in der die Substituenten R¹ und R² die oben genannte Bedeutung haben, **dadurch gekennzeichnet, dass** man als Reduktionsmittel mindestens ein Salz der Dithionigen Säure, der Hydroxymethansulfinsäure oder ein Gemisch aus mindestens einem Salz der Dithionigen Säure und mindestens einem Salz der Hydroxymethansulfinsäure verwendet.

2. Verfahren nach Anspruch 1, **dadurch gekennzeichnet, dass** man als Reduktionsmittel Natriumdithionit verwendet.

3. Verfahren nach Anspruch 2, **dadurch gekennzeichnet, dass** man als Reduktionsmittel ein Gemisch aus Natriumdithionit und einer Base, ausgewählt aus der Gruppe, bestehend aus Natriumcarbonat, Natriumhydrogencarbonat, Kaliumcarbonat und Kaliumhydrogencarbonat verwendet.

4. Verfahren nach Anspruch 1, **dadurch gekennzeichnet, dass** man als Reduktionsmittel Natriumformaldehydsulfoxylat verwendet.

5. Verfahren nach einem der Ansprüche 1 bis 4, **dadurch gekennzeichnet, dass** man die Reduktion in Gegenwart von Wasser durchführt.

6. Verfahren nach einem der Ansprüche 1 bis 5, **dadurch gekennzeichnet, dass** man die Reduktion in einem gegenüber den Cyclohexenderivaten der allgemeinen Formeln I bis IV inerten organischen Lösungsmittel durchführt.

7. Verfahren nach einem der Ansprüche 1 bis 6 zur Herstellung der Cyclohexenverbindungen der Formeln Ia und IIa.

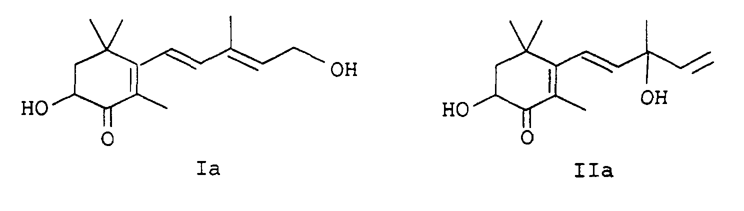

## Claims

1. A process for the preparation of cyclohexene derivatives of general formula I or II: 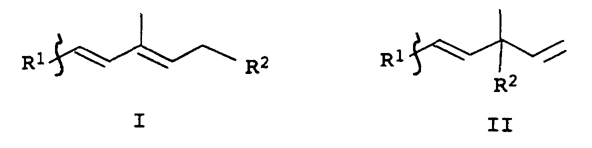 in which the substituents R¹ and R² independently of one another are defined as follows:
R¹ 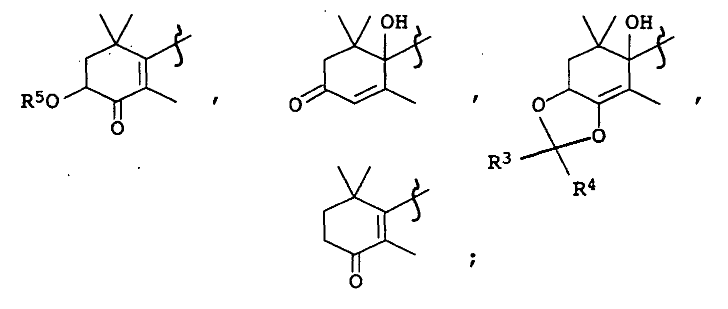
R² is OH or a protecting group convertible to a hydroxyl group by hydrolysis,
R³ and R⁴ are hydrogen or C₁-C₄-alkyl, and
R⁵ is hydrogen or C₁-C₄-acyl,
by the reduction of alkyne compounds of general formula III or IV: 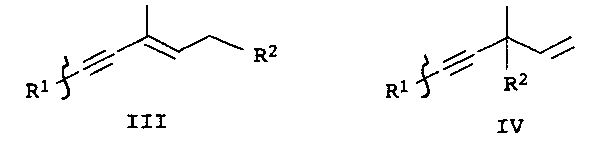 in which the substituents R¹ and R² are as defined above, wherein the reducing agent used is at least one salt of hyposulfurous acid or hydroxymethanesulfinic acid or a mixture of at least one salt of hyposulfurous acid and at least one salt of hydroxymethanesulfinic acid.

2. The process according to claim 1 wherein the reducing agent used is sodium dithionite.

3. The process according to claim 2 wherein the reducing agent used is a mixture of sodium dithionite and a base selected from the group comprising sodium carbonate, sodium hydrogencarbonate, potassium carbonate and potassium hydrogencarbonate.

4. The process according to claim 1 wherein the reducing agent used is sodium formaldehydesulfoxylate.

5. The process according to any of claims 1 to 4 wherein the reduction is carried out in the presence of water.

6. The process according to any of claims 1 to 5 wherein the reduction is carried out in an organic solvent inert towards the cyclohexene derivatives of general formulae I to IV.

7. The process according to any of claims 1 to 6 for the preparation of the cyclohexene compounds of formulae Ia and IIa.

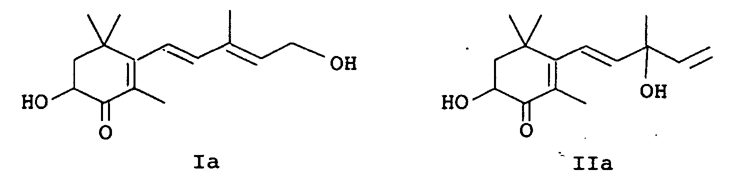

## Revendications

1. Procédé de préparation de dérivés de cyclohexène de la formule générale I ou II : 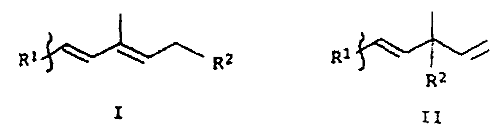 dans lesquelles les substituants R¹ et R² ont, indépendamment l'un de l'autre, la signification suivante :
R¹ représente 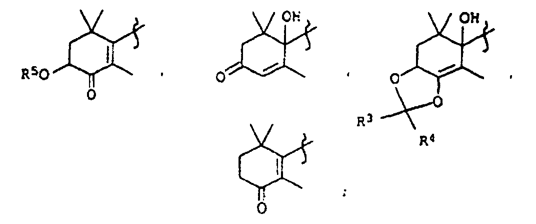
R² représente OH ou un groupe de protection convertissable par hydrolyse en un groupe hydroxy,
R³ et R⁴ représentent de l'hydrogène ou un groupe alkyle en C₁-C₄,
R⁵ représente de l'hydrogène ou un groupe acyle en C₁-C₄,
par réduction de composés alcyniques des formules générales III ou IV : 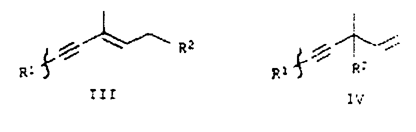 dans lesquelles les substituants R¹ et R² ont la signification indiquée ci-dessus, **caractérisé en ce que**, comme réducteur, on utilise au moins un sel de l'acide hyposulfureux, de l'acide hydroxyméthanesulfinique ou d'un mélange d'au moins un sel de l'acide hyposulfureux et d'au moins un sel de l'acide hydroxyméthanesulfinique.

2. Procédé suivant la revendication 1, **caractérisé en ce que**, comme réducteur, on utilise du dithionite de sodium.

3. Procédé suivant la revendication 2, **caractérisé en ce que**, comme réducteur, on utilise un mélange de dithionite de sodium et d'une base choisie parmi le groupe constitué de carbonate de sodium, de bicarbonate de sodium, de carbonate de potassium et de bicarbonate de potassium.

4. Procédé suivant la revendication 1, **caractérisé en ce que**, comme réducteur, on utilise du formaldéhydesulfoxylate de sodium.

5. Procédé suivant l'une des revendications 1 à 4, **caractérisé en ce qu'**on effectue la réduction en présence d'eau.

6. Procédé suivant l'une des revendications 1 à 5, **caractérisé en ce qu'**on effectue la réduction dans un solvant organique inerte vis-à-vis des dérivés de cyclohexène des formules générales I à IV.

7. Procédé suivant l'une des revendications 1 à 6, pour la préparation des composés de cyclohexène des formules Ia et IIa :
